# FASCICULE DE BREVET EUROPEEN

(11) **EP 3 116 517 B1**
(45) Date de publication et mention de la délivrance du brevet: **04.05.2022**
(21) Numéro de dépôt: 15709153.9
(22) Date de dépôt: 10.03.2015
(51) Int. Cl.: A61K 9/00, A61K 35/74, A61J 3/10, A61K 9/20, A61P 15/02, A61K 9/02, A61P 31/10, A61K 35/747

(54) **COMPRIME VAGINAL MUCO-ADHESIF A LIBERATION PROLONGEE**
MUCOADHÄSIVE VAGINALZUSAMMENSETZUNG MIT VERLÄNGERTER FREISETZUNG
EXTENDED RELEASE MUCOADHESIVE VAGINAL COMPOSITION

(30) Priorité: 10.03.2014 FR 1451951
(43) Date de publication de la demande: 18.01.2017
(73) Titulaire: NEXBIOME THERAPEUTICS, 63000 Clermont-Ferrand (FR)
(72) Inventeur: THORAL, Claudia, F-15500 Massiac (FR); TCHORELOFF, Pierre, F-91440 Bures-sur-Yvette (FR); MAZEL, Vincent, F-33200 Bordeaux (FR); BUSIGNIES, Virginie, F-92340 Bourg-la-Reine (FR); NIVOLIEZ, Adrien, F-15130 Yolet (FR)
(74) Mandataire: Icosa
(86) Numéro de dépôt international: PCT/EP2015/054918
(87) Numéro de publication internationale: WO 2015/135915

(56) Documents cités:
- EP-A1- 1 500 394
- WO-A1-03/026687
- WO-A1-2013/118092
- WO-A2-2013/093941
- EHRSTROM S ET AL: "Lactic acid bacteria colonization and clinical outcome after probiotic supplementation in conventionally treated bacterial vaginosis and vulvovaginal candidiasis", MICROBES AND INFECTION, ELSEVIER, PARIS, FR, vol. 12, no. 10, 1 septembre 2010 (2010-09-01), pages 691-699, XP027276350, ISSN: 1286-4579 [extrait le 2010-05-28]
- FAZELI M R ET AL: "Viability of lactobacillus acidophilus in various vaginal tablet formulations", DARU, TEHRAN UNIVERSITY OF MEDICAL SCIENCES (T UM S) PUBLICATIONS, IR, vol. 14, no. 4, 1 octobre 2006 (2006-10-01), pages 172-177, XP002648806, ISSN: 1560-8115
- MOHAMMED ATHAR ALLI SHEIKH: "Preparation and characterization of a coacervate extended-release microparticulate delivery system for Lactobacillus rhamnosus", INTERNATIONAL JOURNAL OF NANOMEDICINE, 1 août 2011 (2011-08-01), page 1699, XP055145435, DOI: 10.2147/IJN.S19589
- MAGGI L ET AL: "Technological and biological evaluation of tablets containing different strains of lactobacilli for vaginal administration", EUROPEAN JOURNAL OF PHARMACEUTICS AND BIOPHARMACEUTICS, ELSEVIER SCIENCE PUBLISHERS B.V., AMSTERDAM, NL, vol. 50, no. 3, 1 novembre 2000 (2000-11-01), pages 389-395, XP004257218, ISSN: 0939-6411, DOI: 10.1016/S0939-6411(00)00121-1
- Anonymous: "Gy-Na-Tren Vaginal Health Solution by Natren", , 20 octobre 2010 (2010-10-20), XP002730878, Extrait de l'Internet: URL:http://www.rockwellnutrition.com/Gy-Na -Tren-Vaginal-Health-Solution-by-Natren.ht ml#.VDaaa2P4Jos [extrait le 2014-10-09]
- Anonymous: "ProThera - Ther-Biotic", , 24 juin 2009 (2009-06-24), XP002730882, Extrait de l'Internet: URL:https://www.protherainc.com/prod/prodd etail.asp?id=K-TWF [extrait le 2014-10-09]
- FARHAN AHMAD ET AL: "Development and in vitro evaluation of an acid buffering bioadhesive vaginal gel for mixed vaginal infections", ACTA PHARMACEUTICA, vol. 58, no. 4, 1 janvier 2008 (2008-01-01), XP055145552, ISSN: 1330-0075, DOI: 10.2478/v10007-008-0023-2
- VICARIOTTO FRANCO ET AL: "Effectiveness of the association of 2 probiotic strains formulated in a slow release vaginal product, in women affected by vulvovaginal candidiasis: a pilot study.", JOURNAL OF CLINICAL GASTROENTEROLOGY OCT 2012, vol. 46 Suppl, octobre 2012 (2012-10), pages S73-S80, XP009180655, ISSN: 1539-2031
- NADER-MACIAS M E F ET AL: "Advances in the knowledge and clinical applications of lactic acid bacteria as probiotics in the urogenital tract", CURRENT WOMEN'S HEALTH REVIEWS 2008 NL, vol. 4, no. 4, 2008, pages 240-257, XP009180670, ISSN: 1573-4048

## Description

### DOMAINE DE L'INVENTION

La présente invention concerne un comprimé vaginal muco-adhésif à libération prolongée tout particulièrement utile dans le traitement des infections vaginales, des vaginoses, en particulier des candidoses vulvovaginales ou des candidoses vulvovaginales récidivantes.

### ARRIERE PLAN DE L'INVENTION

La candidose vulvovaginale (CVV) est une infection mycosique affectant près de 70-75% des femmes en âge de procréer. Environ 40-50% d'entre elles présenteront une récidive. Entre 5 à 8% des femmes sont quant à elles affectées par une CVV récidivante (survenue d'au moins 4 épisodes par an). L'agent pathogène responsable de ces candidoses est généralement *Candida albicans.*

Chez une femme saine, la flore urogénitale comprend près de 50 espèces différentes de microorganismes. Parmi les microorganismes, 95% de la population sont constitués de diverses souches de lactobacilles, aussi appelés "bacilles de Döderlein" qui sont indicateurs de la flore vaginale saine. Ces lactobacilles jouent un rôle de protection contre les pathogènes par divers mécanismes : production de peroxyde d'hydrogène, production d'acide lactique, production de bactériocines, inhibition de l'adhésion et de l'expansion des pathogènes. En particulier, ces lactobacilles maintiennent un pH acide (en particulier compris entre 4,5 et 5) par la production d'acide lactique à partir du glycogène présent dans le mucus vaginal. Ainsi, la croissance de nombreux pathogènes de la flore vaginale tels que *Gardnerella vaginalis, Prevotella bivia, Atopobium vaginae, Neisseria gonorrhoeae, Mycoplasme, Mobiluncus* et surtout *Candida albicans* est inhibée grâce à cet environnement défavorable à leur développement. La flore vaginale normale est ainsi principalement composée de lactobacilles formant un biofilm protecteur à la surface de la muqueuse. Les lactobacilles spécifiques du vagin sont notamment *Lactobacillus crispatus, Lactobacillus jensenii* et *Lactobacillus vaginalis.* Le développement des candidoses vulvovaginales semble être favorisé par une rupture de l'équilibre de la flore urogénitale observée notamment par la diminution de la population de lactobacilles induisant la colonisation du milieu par un pathogène.

Les CVV, tout particulièrement les CVV récidivantes, affectent négativement le confort de certaines femmes. Leurs traitements engendrent des dépenses de santé importantes et s'avèrent difficiles du fait de la pathogénèse multifactorielle de cette affection (traitements antibiotiques, grossesse, stress...).

Typiquement, les CVV sont traitées au moyen d'actifs antifongiques par traitement systémique ou local. Les traitements proposés s'avèrent contraignants, en particulier les traitements locaux (ovules vaginaux). En effet, la posologie des antifongiques par voie locale est généralement journalière se traduisant par une observance plus faible et donc des risques de récidives accrues. Par ailleurs, les effets secondaires des antifongiques peuvent conduire à une perturbation de la flore vaginale normale, accentuant ainsi les risques de récidive par une destruction de la flore naturelle. Les actifs antifongiques sont également susceptibles d'engendrer des phénomènes d'irritation et de sécheresse vaginale associés à une sensation désagréable de pertes lors de la fonte des ovules vaginaux.

Formulations de comprimés vaginaux comprenant *Lactobacillus acidophilus* et hydroxypropylméthylcellulose (HPMC) sont décrites dans Fazeli, M. R. et al., "Viability of lactobacillus acidophilus in various vaginal tablet formulations", Daru, Teheran University of Medical Sciences (TUMS) Publications, IR, (2006), 14(4):172-177, et dans Maggli, L. et al., Eur. J. Pharm. and Biopharm., (2000), 50(3):389-395. Un besoin existe donc pour la mise à disposition de moyens permettant de traiter efficacement les CVV et d'augmenter le confort des femmes de manière à limiter les problèmes d'observance et ainsi réduire les récidives, tout particulièrement permettant de traiter efficacement les CVV au moyen de probiotiques.

### BREVE DESCRIPTION DE L'INVENTION

La présente invention porte sur un comprimé vaginal muco-adhésif à libération prolongée essentiellement constitué de 10⁹ à 10¹¹ UFC d'au moins une souche probiotique du genre *Lactobacillus* par gramme de comprimé comprimée avec de 10 à 30% en poids d'un excipient approprié pour conférer au comprimé ses propriétés de muco-adhésion à la paroi vaginale et de libération prolongée, ledit excipient étant constitué d'hydroxypropylméthylcellulose de viscosité dynamique supérieure à 10 000 mPa.s, et l'au moins une souche probiotique et ledit excipient étant comprimés avec une contrainte de compression allant de 50 à 400 MPa. L'excipient est présent en une quantité qui permet à la fois une bonne adhésion aux muqueuses et une libération prolongée de la souche. La libération prolongée doit répondre aux deux exigences d'assurer la viabilité de la souche et de permettre une administration espacée dans le temps du comprimé. On recherche en effet un traitement qui permette une administration d'au plus un comprimé tous les deux jours, avantageusement au plus tous les 3 jours ou plus.

La présente invention porte également sur un procédé de préparation d'un comprimé vaginal muco-adhésif à libération prolongée comprenant les étapes suivantes :
a. Mélange d'au moins 10⁹ à 10¹¹ UFC par gramme de comprimé d'au moins une souche probiotique du genre *Lactobacillus* avec 10 à 30 % en poids, par rapport au poids total du comprimé de l'hydroxypropylméthylcellulose appropriée pour conférer au comprimé ses propriétés de muco-adhésion à la paroi vaginale et de libération prolongée ;
b. Compression du mélange obtenu à l'étape a. avec une contrainte de compression allant de 50 à 400 MPa.

La présente invention porte également sur ce comprimé pour une utilisations dans le traitement des infections vaginales, des vaginoses.

### BREVE DESCRIPTION DES FIGURES

La figure 1 représente les résultats de l'étude de la libération de la souche Lcr35^{®} à partir de la poudre commerciale (*Lcr regenerans*^{®}*,* carrés) et du comprimé selon la présente invention (comprimé LP, losange).
La figure 2 représente l'évolution en termes d'hydratation et d'érosion du comprimé de la présente invention dans le milieu SVF au cours du temps (96h).
La figure 3 représente l'évolution de la viabilité de la souche Lcr35^{®} (In) en fonction du temps (mois) en condition de stabilité accélérée selon le référentiel ICH (40°C) pour une poudre libre (croix, ligne continue pour l'extrapolation linéraire) et pour les comprimés contenant la souche formulée (*Lcr regenerans*^{®}) obtenus à différentes contraintes de compressions (100MPa - triangle, 200 MPa - losange, 300 MPa - signe plus, 400 MPa - tiret).
La figure 4 représente les résultats de l'étude de la croissance de la souche Lcr35^{®} à partir du comprimé selon la présente invention : UFC dans le milieu à différents temps (exprimés en heure).
La figure 5 représente les résultats de l'étude d'inhibition de la croissance de C. *albicans* par la souche Lcr35^{®} issue du comprimé selon la présente invention en contact direct : viabilité (UFC.mL⁻¹) à différents temps (exprimés en heure) pour le témoin (*C*. *albicans* ; gris) et le comprimé selon la présente invention (*C*. *albicans* + comprimé LP ; noir).
La figure 6 représente les résultats de l'étude d'inhibition de la croissance de *G*. *vaginalis* par la souche Lcr35^{®} issue du comprimé selon la présente invention en contact direct ou après une phase de pré-culture : viabilité (UFC.ml⁻¹) en fonction du temps (heures) (noir : témoin G. *vaginalis,* hachures : *G. vaginalis* + comprimé LP contact direct; croisillons : *G. vaginalis* + comprimé LP pré-culture).

### DEFINITIONS

Le terme « UFC » désigne l'unité de mesure reconnue par l'homme du métier pour quantifier les bactéries capables de fonder une colonie et signifie précisément « Unité Formant Colonie ».

Le terme API signifie « Active Pharmaceutical Ingrédient » également appelé substance active.

Le terme « probiotique » désigne des microorganismes vivants qui administrés en quantité suffisante confèrent un effet bénéfique sur la santé de l'hôte.

L'expression « propriétés de muco-adhésion à la paroi vaginale » se définit comme étant l'adhésion de deux surfaces dont l'une est un épithélium. Les propriétés de muco-adhésion à la paroi vaginale peuvent être déterminées par différentes méthodes, telles que décrites dans la référence European Journal of Pharmaceutics and Biopharmaceutics, 85, (2013) 843-853. En particulier, les propriétés de muco-adhésion à la paroi vaginale peuvent être évaluées en déterminant le temps ou la force nécessaire pour détacher une forme galénique d'une muqueuse.

L'expression « comprimé à libération prolongée » désigne un comprimé dans lequel la vitesse de libération de la substance active est ralentie, la libération de la substance active étant étalée dans le temps (par opposition à une libération immédiate « flash »). De préférence, l'expression « comprimé à libération prolongée » désigne un comprimé assurant la diffusion du principe actif sur une durée plus longue que la libération conventionnelle souvent assimilée à une dose flash.

### DESCRIPTION DETAILLEE DE L'INVENTION

Les inventeurs ont mis au point une forme galénique incluant des probiotiques permettant de combiner efficacité de traitement et confort des femmes lors de la prise des probiotiques conduisant avantageusement à une augmentation de l'observance du traitement des infections vaginales, des vaginoses, en particulier des CVV, et ainsi évitant leurs récidives.

L'invention porte donc sur un comprimé vaginal muco-adhésif à libération prolongée essentiellement constitué de 10⁹ à 10¹¹ UFC d'au moins une souche probiotique du genre *Lactobacillus* par gramme de comprimé comprimée avec de 10 à 30% en poids d'un excipient approprié pour conférer audit comprimé ses propriétés de muco-adhésion à la paroi vaginale et de libération prolongée, ledit excipient étant constitué d'hydroxypropylméthylcellulose de viscosité dynamique supérieure à 10 000 mPa.s, l'au moins une souche probiotique et ledit excipient étant comprimés avec une contrainte de compression allant de 50 à 400 MPa. Le comprimé vaginal muco-adhésif à libération prolongée peut comprendre une ou plusieurs souches probiotiques du genre *Lactobacillus.*

La forme galénique mise au point par les inventeurs permet une libération prolongée d'une souche lactobacillaire tout en intégrant des propriétés de muco-adhésion sur la paroi vaginale, limitant les désagréments chez la femme, et de libération prolongée permettant ainsi d'outrepasser les problèmes de posologie et de risque de récidives (associées à la résistance du pathogène). Le comprimé de la présente invention est destiné à une administration locale. L'application pourra être effectuée par la patiente ou bien à l'aide d'un applicateur adapté à la forme du comprimé pour en faciliter l'administration.

L'administration de souches probiotiques du genre *Lactobacillus* est connue pour promouvoir la santé vaginale. En particulier, des souches probiotiques du genre *Lactobacillus* utiles pour traiter les infections vaginales, les vaginoses, en particulier les CVV et prévenir leurs récidives peuvent être telles que décrites dans WO 2014/009349.

Le comprimé vaginal muco-adhésif à libération prolongée selon la présente invention comprend de préférence une souche probiotique du genre *Lactobacillus* choisie parmi *Lactobacillus rhamnosus, Lactobacillus crispatus, Lactobacillus iners, Lactobacillus gasseri, Lactobacillus jensenii, Lactobacillus acidophilus, Lactobacillus plantarum, Lactobacillus vaginalis, Lactobacillus casei, Lactobacillus reuteri* et leurs combinaisons, en particulier deux à deux. De préférence, la souche probiotique du genre *Lactobacillus* est Lcr35^{®} (*Lactobacillus rhamnosus*)*.* Dans une autre variante, la souche probiotique du genre *Lactobacillus* est la souche *Lactobacillus crispatus IP174178 déposée* à *la CNCM sous le numéro I-4646.* Dans une autre variante, on utilise une combinaison de *Lactobacillus crispatus* et *Lactobacillus rhamnosus* (avantageusement Lcr35^{®}).

Le comprimé vaginal muco-adhésif à libération prolongée selon la présente invention comprend de 10⁹ à 10¹¹ UFC de la souche probiotique du genre *Lactobacillus* par gramme de comprimé après fabrication (teneur mesurée le jour de la fabrication). Typiquement, le comprimé vaginal muco-adhésif à libération prolongée selon la présente invention comprend au moins 10⁷ UFC de la souche probiotique du genre *Lactobacillus* souches par gramme de comprimé après stockage pendant 36 mois à 25°C. Lorsqu'une combinaison de souches est utilisée, chacune de ces souches est présente à de telles concentrations.

De manière avantageuse, le comprimé selon la présente invention permet de conserver la dose de la souche probiotique tout en étalant la libération de cette dose dans le temps. Ceci permet de diminuer la posologie pour améliorer l'observance des patientes tout en allongeant la durée de l'effet thérapeutique afin d'éviter une récidive.

La souche probiotique du genre *Lactobacillus* est de préférence sous forme lyophilisée. Avant lyophilisation, la souche est avantageusement mélangée avec des cryoprotecteurs pour lui assurer une très grande stabilité à température ambiante. Par exemple, la souche peut être mélangée avec un thiosulfate de métal alcalin ou alcalino-terreux (FR 1 426 226), avantageusement le thiosulfate de sodium.

Le comprimé vaginal muco-adhésif à libération prolongée selon la présente invention comprend de l'hydroxypropylméthylcellulose en tant qu'excipient approprié pour conférer au comprimé ses propriétés de muco-adhésion à la paroi vaginale et de libération prolongée. La présente description décrit également d'autres excipients appropriés pour conférer au comprimé ses propriétés de muco-adhésion à la paroi vaginale et de libération prolongée, qui sont connus de l'homme du métier. Des exemples d'excipients pouvant convenir sont décrits dans Advanced Drug Delivery System Reviews, 57 (2005), 1692-1712. Il est décrit que l'excipient peut être choisi parmi le chitosan et ses dérivés, les pectines, les polyéthylènes glycols, l'alginate de sodium, les acides polyacryliques, les dérivés cellulosiques tels que la carboxyméthylcellulose sodique, l'hydroxypropylméthylcellulose ou la cellulose microcristalline, les gommes, les Carbopol^{®} et leurs combinaisons.

Dans le cadre de l'invention, on utilise de l'hydroxypropylméthylcellulose de haute viscosité. Ainsi, on utilise de l'hydroxypropylméthylcellulose ayant une viscosité dynamique supérieure à 10 000 mPa.s, avantageusement comprise entre 11 000 mPa.s et 21 000 mPa.s, plus avantageusement d'environ 15 000 mPa.s.

La viscosité de cet excipient bien connue est mesurée selon la norme européenne ou américaine pour une solution aqueuse à 2% (m/v) d'hydroxypropylméthylcellulose à 20°C.

Le comprimé vaginal muco-adhésif à libération prolongée selon la présente invention comprend généralement de 10 à 30 % en poids par rapport au poids total du comprimé d'un excipient conférant au comprimé ses propriétés de muco-adhésion à la paroi vaginale et de libération prolongée. De préférence, le comprimé comprend de 10 à 25 % en poids par rapport au poids total du comprimé dudit excipient, plus avantageusement de 10 à 20% en poids, voire il comprend 10 % en poids dudit excipient, ledit excipient étant de l'hydroxypropylméthylcellulose de viscosité dynamique supérieure à 10 000 mPa.s.

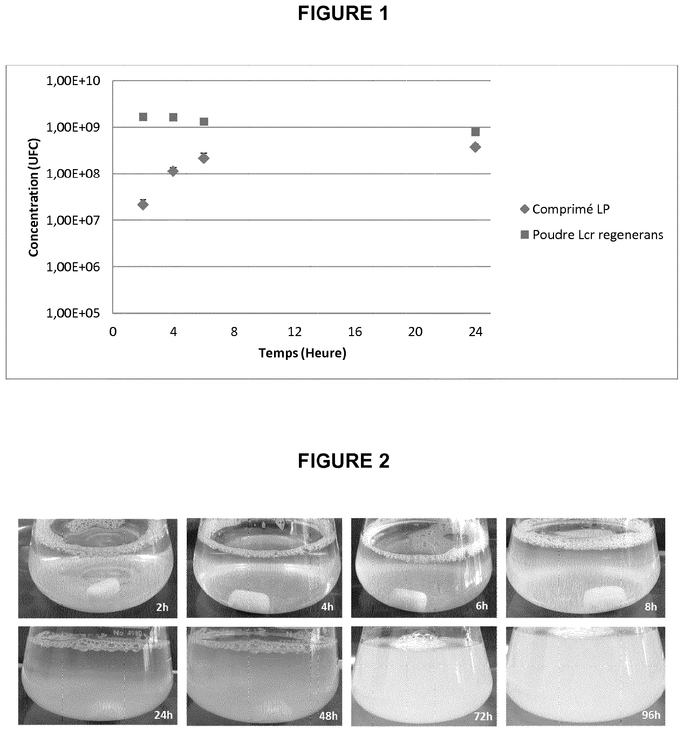

De manière avantageuse, les excipients tels que décrits ci-dessus permettent une libération prolongée dans le temps de la souche probiotique. En particulier, il a été montré que le comprimé de la présente invention permet une libération progressive de la souche contrairement à la libération immédiate de la souche dans un milieu à partir de la poudre commerciale (à posologie équivalentes) (Figure 1), la poudre commerciale étant la poudre contenue dans les gélules (gélules, *Lcr regenerans*^{®}) commercialisées sous le nom de Gynophilus^{®} par la société PROBIONOV.

Plus précisément, il a été montré que le comprimé de la présente invention permet d'aboutir à des quantités de bactéries équivalentes à la poudre commerciale au niveau du plateau de libération et cela pour une quantité initiale de bactéries dans la forme pharmaceutique de l'ordre de 10⁹ UFC/g. D'autre part, il a été montré que l'hydratation et l'érosion du comprimé s'étalent sur au moins 72h permettant une prolongation de la libération de la souche Lcr35^{®} (Figure 2). Ainsi, le comprimé vaginal muco-adhésif à libération prolongée selon la présente invention peut être administré tous les 3-7 jours, soit un comprimé tous les 3, 4, 5, 6 ou 7 jours. Typiquement, les gélules sont administrées une fois par jour. L'administration du comprimé de la présente invention permet donc de diminuer les prises médicamenteuses et ainsi, d'améliorer l'observance des patientes.

Selon une variante avantageuse de l'invention le comprimé est destiné à une administration locale au plus tous les 2 jours, avantageusement au plus tous les 3 jours, ou tous les 4 jours, ou tous les 5 jours, ou tous les 6 jours ou tous les 7 jours.

La souche probiotique du genre *Lactobacillus,* de préférence sous forme lyophilisée, et l'hydroxypropylméthylcellulose de viscosité dynamique supérieure à 10 000 mPa.s conférant au comprimé ses propriétés de muco-adhésion à la paroi vaginale et de libération prolongée sont comprimées avec une contrainte de compression allant de 50 à 400 MPa pour donner le comprimé de la présente invention. De préférence, la souche probiotique et l'hydroxypropylméthylcellulose sont comprimés par une contrainte de compression allant de 200 à 250 MPa. De manière préférée, la contrainte de compression est de 250 MPa.

Il est bien connu qu'il est nécessaire de maîtriser la stabilité de toute formulation pharmaceutique comprenant une souche probiotique au cours de sa durée de vie (de sa fabrication à sa consommation) pour que l'effet thérapeutique attendu puisse être observé. Il est également connu que la viabilité des bactéries est sensible aux procédés de fabrication (et notamment lors de l'étape de compression) et aux conditions et durées de stockage.

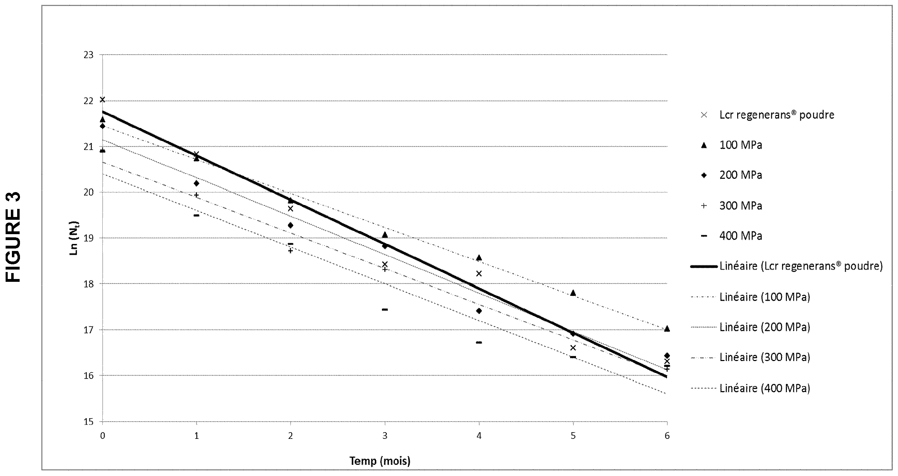

De manière inattendue, les inventeurs ont mis en évidence que le stress subi par la souche probiotique lors de la compression n'affecte pas négativement la stabilité (viabilité) de la souche en conditions accélérées de stockage (40°C). En limitant la surface d'échange avec le milieu extérieur, la compression permet même d'augmenter la stabilité de la souche par comparaison avec la poudre libre (taux de destructions plus faibles pour les comprimés par rapport à la poudre libre) (Figure 3).

Ainsi, les comprimés de la présente invention peuvent garantir l'administration d'une dose utile de probiotique et garantir ainsi l'observation de l'effet thérapeutique recherché.

Le comprimé vaginal muco-adhésif à libération prolongée selon la présente invention peut en outre comprendre des excipients pharmaceutiquement acceptables bien connus de l'homme du métier. En particulier, le comprimé peut comprendre des cryoprotecteurs, conservateurs, lubrifiants, agents d'écoulement, liants, diluants et leurs combinaisons. Par exemple, le comprimé peut comprendre un ou plusieurs des excipients suivants : inuline et ses dérivés, maltodextrine, dextrose et ses dérivés, fructo-oligosaccharide, amidon, tréalose, glucose, stéarate de magnésium.

Le comprimé vaginal muco-adhésif à libération prolongée selon la présente invention peut en outre comprendre des principes actifs ayant une action complémentaire. En particulier, le comprimé peut comprendre des hormones (estriol, progestérone...), des agents antiinflammatoires, des agents bactéricides et/ou des agents antifongiques. L'homme du métier saura déterminer quels principes actifs peuvent être avantageusement combinés avec une souche probiotique du genre *Lactobacillus.*

Le comprimé vaginal muco-adhésif à libération prolongée selon la présente invention peut être utilisé dans la prévention et/ou le traitement des infections vaginales.

Le comprimé vaginal muco-adhésif à libération prolongée selon la présente invention peut être utilisé dans la prévention et/ou le traitement des vaginoses.

Le comprimé vaginal muco-adhésif à libération prolongée selon la présente invention peut être utilisé dans la prévention et/ou le traitement des candidoses.

Le comprimé vaginal muco-adhésif à libération prolongée selon la présente invention peut être utilisé dans la prévention et/ou le traitement des candidoses vulvovaginales et des candidoses vulvovaginales récidivantes.

Le cas échéant, le comprimé vaginal muco-adhésif à libération prolongée selon la présente invention peut être administré en combinaison avec un traitement antibiotique ou antifongique, ou consécutivement à un tel traitement.

Ainsi, dans un aspect, la présente invention porte sur un comprimé tel que décrit dans les revendications pour une utilisation dans une méthode de prévention et/ou de traitement des infections vaginales, des vaginoses, des candidoses qui peuvent être récidivantes, en particulier des candidoses vulvovaginales ou candidoses vulvovaginales récidivantes, comprenant l'administration à une femme, par voie locale, d'un comprimé vaginal muco-adhésif à libération prolongée comprenant une souche probiotique du genre *Lactobacillus* comprimée avec un excipient approprié pour conférer audit comprimé ses propriétés de muco-adhésion à la paroi vaginale et de libération prolongée. Le composé peut être administré une fois tous les 3-7 jours, soit un comprimé tous les 3, 4, 5, 6 ou 7 jours. Selon les indications (traitement de confort, traitement d'une récidive ou traitement d'une récidive et rééquilibration de la flore), l'administration pourra être renouvelée deux, quatre ou six fois. Les infections vaginales, les candidoses et les vaginoses peuvent être toute infection liée à la croissance pathologique ou déséquilibrée de nombreux parasites ou pathogènes de la flore vaginale tels que ceux du genre *Atopobium, Candida, Gardnerella, Neisseria, Mycoplasma, Mobiluncus, Prevotella, Trichomonas. On peut en particulier citer les espèces suivantes : Atopobium vaginae, Candida albicans, Gardnerella vaginalis, Neisseria gonorrhoeae, Mycoplasma hominis, Mycoplasma genitalium, Mobiluncus, Prevotella bivia, Trichomonas vaginalis.* Le pathogène peut être *Candida albicans.* Le pathogène peut être *Gardnerella vaginalis.*

La vaginose, ou vaginose bactérienne, est un déséquilibre de la flore microbienne du vagin. Elle se caractérise par la disparition des lactobacilles et la multiplication de germes anaérobies tels que le *Gardnerella vaginalis.* Elle témoigne d'un déséquilibre de la flore vaginale.

La présente invention porte également sur un procédé de préparation d'un comprimé vaginal muco-adhésif à libération prolongée comprenant les étapes suivantes :
a. Mélange d'au moins 10⁹ à 10¹¹ UFC par gramme de comprimé d'au moins une souche probiotique du genre *Lactobacillus* avec 10 à 30 % en poids, par rapport au poids total du comprimé, d'hydroxypropylméthylcellulose appropriée pour conférer audit comprimé ses propriétés de muco-adhésion à la paroi vaginale et de libération prolongée, optionnellement des excipients pharmaceutiquement acceptables et/ou principes actifs ayant une action complémentaire ;
b. Compression du mélange obtenu à l'étape a. avec une contrainte de compression allant de 50 à 400 MPa pour obtenir d'un comprimé vaginal muco-adhésif à libération prolongée.

La souche probiotique, l'hydroxypropylméthylcellulose conférant les propriétés de muco-adhésion à la paroi vaginale et de libération prolongée, les principes actifs ayant une action complémentaire et les excipients pharmaceutiquement acceptables sont tels que décrits précédemment.

### EXEMPLES

### Etude de la viabilité de la souche Lcr35^{®}

Des comprimés de 650 mg contenant la souche Lcr35^{®} (API *Lcr regenerans*^{®}*,* 100% en poids) ont été fabriqués à 4 contraintes de compression différentes : 100, 200, 300 et 400 MPa puis placés dans des piluliers en verre fermés hermétiquement. Des piluliers contenant la poudre libre (témoin) ont également été suivis.

Selon le référentiel ICH relatif au suivi des stabilités des produits pharmaceutiques, le suivi de la stabilité de la souche a été effectué en conditions accélérées (40°C) afin d'évaluer le taux de destruction associé à chaque contrainte de compression.

Les points de contrôles ont été effectués à 0, 1, 2, 3, 4, 5 et 6 mois. Les résultats obtenus sont exprimés en UFC/g. A partir de ces données, le graphique de la figure 3 a été obtenu par le calcul du logarithme népérien de Nₜ (avec Nₜ la quantité de bactéries présentes au temps t).

Les taux de destruction sont obtenus grâce à la pente de la droite représentant In(Nₜ/N₀) en fonction du temps.

Les taux de destruction associés à l'étude de stabilité en conditions accélérées pour la poudre et les comprimés sont présentés dans le tableau ci-dessous :

| **Contrainte de compression** (MPa) | **Taux de destruction k (R²)** |
|---|---|
| | *Lcr regenerans*^{®} |
| **0** | **1.0274 (0.97)** |
| **100** | 0.7727 (0.99) |
| **200** | 0.9058 (0.97) |
| **300** | 0.8367 (0.97) |
| **400** | 0.9124 (0.91) |

Les taux de destruction associés aux contraintes de compression démontrent un impact plus important des conditions de stockage sur la poudre (taux de destruction >1,0) par rapport aux comprimés fabriqués aux différentes contraintes de compression (taux de destructions <0,9). La compression n'a pas affecté de manière néfaste la capacité de la souche à se maintenir dans des conditions de stockage à 40°C. Les résultats obtenus démontrent que la stabilité de la souche est même améliorée après compression en conditions de stabilité accélérées (diminution de la surface d'échange).

### Préparation du comprimé LP

Le comprimé LP est préparé par mélange de 900 mg d'API *Lcr regenerans*^{®} et 100 mg d'HPMC (Metolose^{®} 90SH-15000SR) et comprimé avec une contrainte de compression de 250 MPa.

### Libération de la souche Lcr35^{®}à partir de la poudre commerciale (Lcr regenerans^{®}) et du comprimé selon la présente invention (comprimé LP)

Le terme « poudre commerciale » désigne l'API *Lcr regenerans*^{®}*.* Des gélules comprenant l'API *Lcr regenerans*^{®} sont disponibles commercialement (Gynophilus^{®}).

250 ml de milieu SVF (Simulated Vaginal Fluid) ajusté à pH 4,2 sont préparés dans un erlenmeyer pour l'essai. L'échantillon est introduit dans le milieu à T₀. L'erlenmeyer est ensuite placé sur une plaque d'agitation à 75 rpm elle-même placée dans une étuve à 37°C.

A intervalles réguliers, 1ml de milieu est prélevé pour réaliser la quantification des bactéries présentes dans le SVF (dénombrement sur gélose MRS) puis 1ml de milieu frais est replacé. Les résultats obtenus après un suivi de 24 heures sont présentés à la figure 1.

Un suivi visuel du comprimé a également été effectué sur cet essai (96h) afin d'évaluer l'état de l'unité dans le milieu. L'érosion progressive du comprimé de la présente invention est évaluée en heure. Une érosion complète est définie par la disparition de la forme comprimé pour l'obtention d'une suspension.

Après mise en contact avec le milieu SVF de 900 mg de poudre commerciale (quantité équivalente à la quantité d'API présente dans le comprimé LP), une mise à disposition immédiate des bactéries dans le milieu est observée. La quantité administrée au T₀ est retrouvée à tous les temps de prélèvements. Ceci démontre la libération immédiate et totale de la souche à partir de la forme gélule une fois la gélule dissoute.

En comparaison, le comprimé LP placé dans le même milieu libère progressivement la souche et ce de manière contrôlée (figure 1). Cette libération dite contrôlée s'évalue par l'augmentation de la quantité des bactéries dans le milieu sur les premières heures de suivi (2, 4, 6 et 24h) pour atteindre un maximum équivalent à la poudre après 24h. La libération de la souche à partir de la forme « comprimé » est contrôlée sur les premières heures afin de coloniser progressivement le milieu pour atteindre une valeur seuil (24h) équivalente à la poudre libre à posologie égale. La valeur seuil est de l'ordre de 10⁹ UFC dans le milieu.

Le comprimé conserve son intégrité pendant 24h en formant une matrice polymérique au travers de laquelle, la souche Lcr35^{®} est libérée. Ensuite, une érosion progressive de l'unité est observée. La libération est ainsi prolongée jusqu'à l'érosion totale du comprimé qui est observée après 72h d'incubation dans le SVF. Le milieu est ainsi saturé en *lactobacillus* permettant d'obtenir un effet thérapeutique sur plusieurs jours conduisant à une diminution de la posologie.

Dans le cadre de la présente invention, un comprimé peut être considéré « à libération prolongée » lorsqu'est observée une libération progressive de la souche probiotique (par opposition à une libération flash). Tout particulièrement, un comprimé peut être considéré « à libération prolongée » lorsqu'une libération progressive de la souche probiotique est observée lors de la mise en œuvre de la méthode décrite ci-dessus. Une libération peut être considérée progressive lorsqu'elle croit sur une plage d'au moins deux heures lors de la mise en œuvre de la méthode décrite ci-dessus.

### Capacités de croissance de la souche Lcr35^{®}libérée du comprimé LP

Un mini-fermenteur (500 ml) est utilisé comme enceinte close pour évaluer la croissance de la souche.

Le milieu utilisé est le MRS qui est préalablement ajusté à pH 4,2 (pH vaginal). Après stérilisation du montage expérimental, un comprimé LP est placé dans le milieu MRS (250 ml) qui est maintenu à 37°C et agité à 200 rpm (hélice). Le suivi de la croissance s'effectue par prélèvements réguliers du milieu pour un dénombrement après dilution sur gélose MRS. Le volume prélevé est remplacé par du milieu frais après chaque prélèvement.

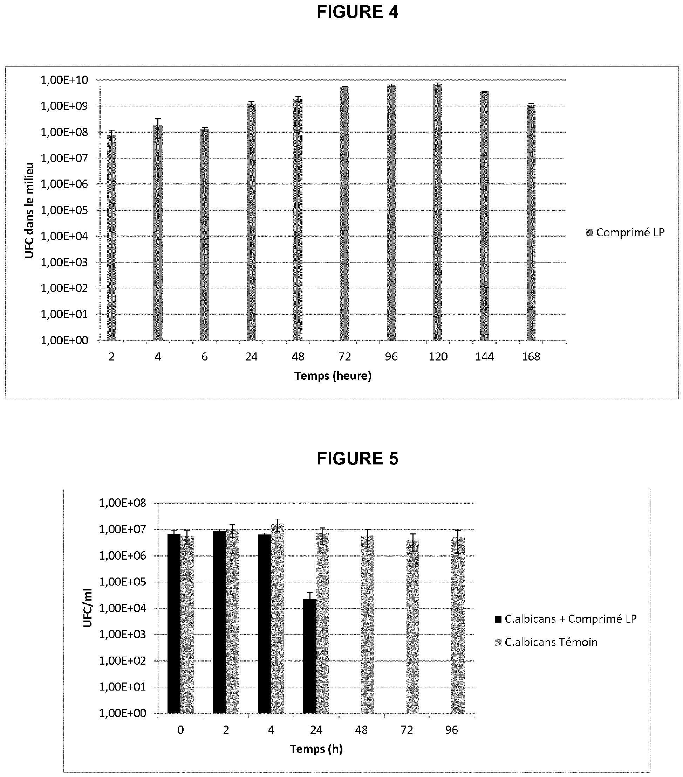

Les résultats sont présentés à la figure 4. Ils démontrent la capacité de la souche à se régénérer et à croître une fois libérée du comprimé et ce, jusqu'à des valeurs proches de 10¹⁰ UFC dans le milieu. Cette étude confirme qu'en plus d'être libérée du comprimé (cf. ci-dessus), la souche conserve sa capacité de multiplication et de colonisation une fois en contact avec un milieu favorable à son développement. La colonisation du milieu par la souche est un élément indispensable à l'obtention de l'effet thérapeutique sur les CVV et les CVV récidivantes mais également à la restauration d'une flore vaginale saine.

### Inhibition de la croissance des souches pathogènes C. albicans et G. vaginalis en contact avec le comprimé LP

Un certain nombre d'études *in vitro* et *in vivo* ont démontré la capacité du *Lactobacillus rhamnosus* Lcr35^{®} :
- à s'implanter dans l'épithélium vaginal, ainsi que la longévité d'une telle implantation (Coudeyras S, Jugie G, Vermerie M, Forestier C. Adhésion of human probiotic Lactobacillus rhamnosus to cervical and vaginal cells and interaction with vaginosis and vaginitis-associated pathogens. 2008. Infectious diseases in obstetrics and gynecology ; Coudeyras S, Jugie G, Vermerie M, Forestier C. Adhesion of the probiotic Lactobacillus Lcr35 to immortalized human cervical and vaginal epithelial cells, Infect Dis Obstet Gynecol. 2008; 2008: 549640) ;
- et à inhiber la croissance de germes pathogènes responsables de vaginoses bactériennes (*Gardnerella vaginalis* et *Prevotella bivia*) mais aussi de mycoses (*Candida albicans*) en produisant diverses substances microbicides (Coudeyras S, Jugie G, Vermerie M, Forestier C. Adhesion of human probiotic Lactobacillus rhamnosus to cervical and vaginal cells and interaction with vaginosis and vaginitis-associated pathogens. 2008. Infectious diseases in obstetrics and gynecology; A. Nivoliez, O. Camares, M. Paquet-Gachinat, S. Bornes, C. Forestier, P. Veisseire, Influence of manufacturing processes on in vitro properties of the probiotic strain Lactobacillus rhamnosus Lcr35®, J. Biotechnol. 160 (2012) 236-241).

Le but de développer une telle forme pourrait permettre de traiter les infections vaginales autant en curatif que préventif comme les vaginoses et plus particulièrement les CVV. Pour cela, la souche doit pouvoir agir sur la souche pathogène une fois libérée du comprimé.

Ici, la capacité d'inhibition est testée contre la souche pathogène: *C. albicans.* Le comprimé LP de ladite invention est mis en contact direct avec une culture de *C. albicans.*

Pour cela, une pré-culture de la souche pathogène *C. albicans* est réalisée dans du milieu Sabouraud (AES, Bruz, France) à 25°C. En proportions équivalentes, la pré-culture est mise en contact avec un milieu MRS (Biomérieux, Marcy l'étoile, France) contenant un comprimé LP. Un deuxième pilulier est suivi sans la présence du comprimé pour évaluer la croissance de la souche pathogène seule (*C. albicans* témoin).

Le suivi s'est effectué sur 96h à 37°C. A chaque point de contrôle (0, 2, 4, 24, 48, 72, 96h), un dénombrement de la souche pathogène est réalisé sur gélose Sabouraud incubée à 25°C pendant 5 jours.

Les résultats obtenus sont présentés à la figure 5. Le contact du comprimé LP avec une culture de *C. albicans* permet d'inhiber significativement la croissance du pathogène au bout de 24h conduisant à son inhibition complète après 48h de co-culture. La souche Lcr35^{®} libérée à partir du comprimé LP conserve donc ses capacités d'inhibition du pathogène. En ayant une libération prolongée de la souche Lcr35^{®} et donc une action prolongée contre la croissance du pathogène, la souche libérée du comprimé pourra ensuite se réimplanter dans la cavité vaginale pour un retour à l'équilibre naturel de la flore.

### Inhibition de la croissance de la souche pathogène G. vaginalis en contact avec le comprimé LP

La concentration du pathogène (*G. vaginalis)* seul et en présence de la souche *Lactobacillus rhamnosus* Lcr35 conditionnée en comprimé à libération prolongée a été évaluée avec détermination du titre à T0, T4h, T6h, T8h, T24h, T30h. La capacité d'inhibition du comprimé LP a été évalué par contact direct (culture préalable du pathogène et ajout d'un comprimé LP) et suite à une étape de pré-culture pour le pathogène et pour la souche contenue dans le comprimé.

Les Milieux de culture utilisés pour la préparation des inocula et la lecture des titres sont :
- bouillon BHI/gélose gardnerella pour *Gardnerella vaginalis*
- bouillon MRS/gélose MRS pour les souches de Lactobacilles
- le bouillon de culture de l'essai est un mélange homogène du milieu de culture du pathogène et de MRS.

Après la mise en contact et à chaque point de prélèvement, 1 ml est prélevé et les dilutions successives aux 10ème sont ensuite effectuées dans des tubes d'eau stériles. Aux dilutions adéquates, 1ml est incubé dans une gélose MRS à 37°C pendant 3 jours pour la souche Lcr35 et 0,1 ml est ensemencé en surface sur gélose *Gardnerella* et placé à 37°C pendant 48h en présence de CO₂ pour *G. vaginalis.*
- Pour la fabrication du témoin *Gardnerella vaginalis,* on place 15 ml de bouillon BHI inoculé avec la souche pathogène et 15 ml de bouillon MRS non inoculé.
- Pour la mise en contact des inocula onplace 15 ml de la souche pathogène avec 15 ml de bouillon MRS inoculé (pré-culture) ou bien non inoculé où l'on place un comprimé LP (contact direct).
- Points de suivi : On réalise les prélèvements et les déterminations du titre à T0, T4h, T6h, T8h, T24h et T30h.

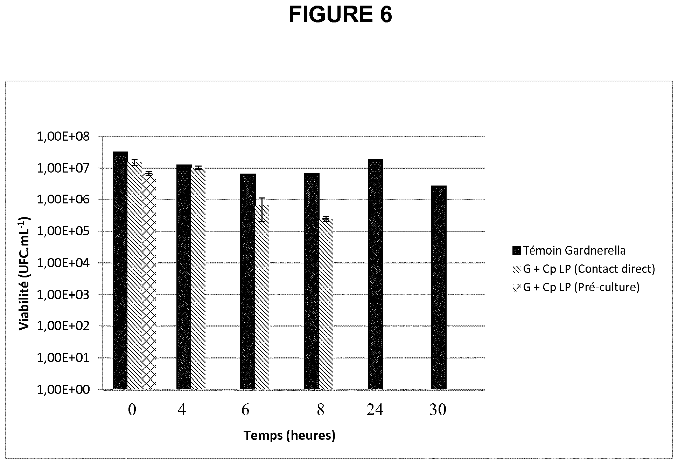

Les résultats obtenus sont présentés à la figure 6. Le contact du comprimé LP avec une culture de *G. vaginalis* permet d'inhiber significativement la croissance du pathogène au bout de 6h conduisant à son inhibition complète après 24h de co-culture. La souche Lcr35^{®} libérée à partir du comprimé LP conserve donc ses capacités d'inhibition du pathogène. En ayant une libération prolongée de la souche Lcr35^{®} et donc une action prolongée contre la croissance du pathogène, la souche libérée du comprimé pourra ensuite se réimplanter dans la cavité vaginale pour un retour à l'équilibre naturel de la flore.

### Muco-adhésion du comprimé LP

Des essais ont été réalisés sur des parois vaginales de porc fraîchement excisées. Le protocole est le suivant.

### ✔ Mise en place de la muqueuse vaginale et du comprimé

Les essais sont effectués sous un Poste de Sécurité Microbiologique de manière stérile. Le milieu SVF pH 4,2 est préchauffé à 37°C (50 ml par échantillon).

Une portion de muqueuse vaginale permettant de recouvrir le fond d'un flacon stérile est prélevée. Le fond du flacon est recouvert avec de la colle cyanoacrylate puis la muqueuse est disposée de manière à obtenir une surface uniforme. La muqueuse est mouillée avec du SVF pH 4,2 (environ 1ml) puis, le comprimé est placé sur la muqueuse en exerçant une pression avec le doigt (20 secondes).

Sont ensuite versés lentement dans le flacon les 50 ml de SVF préchauffé, qui est ensuite refermé.

### ✔ Essai de muco-adhésion dans le SVF pH 4,2

Le flacon est placé sur un banc d'agitation à 75 rpm, à 37°C, jusqu'à détachement du comprimé de la surface vaginale. Le suivi de la muco-adhésion s'effectue toutes les 30 minutes pendant 7h puis, à partir de 24h, toutes les heures jusqu'à son détachement ou son érosion complète. Le pouvoir muco-adhésif du comprimé est exprimé en heure.

Ce protocole a été appliqué au comprimé LP de ladite invention ainsi qu'à la gélule commerciale. Les résultats ont montrés que la gélule présente une muco-adhésion très limitée (2h±17min) tandis que le comprimé LP muco-adhésif adhère à la paroi sur une durée de 21h±1h. L'érosion complète du comprimé est également observée au bout de 72h d'incubation (figure 2). Le paramètre de muco-adhésion est un paramètre fondamental afin de maintenir la forme dans la cavité sur un temps suffisamment long pour permettre l'implantation de la souche dans la cavité vaginale puis l'inhibition du pathogène.

## Revendications

1. Comprimé vaginal muco-adhésif à libération prolongée, **caractérisé en ce qu'**il est essentiellement constitué de 10⁹ à 10¹¹ UFC d'au moins une souche probiotique du genre *Lactobacillus* par gramme de comprimé comprimée avec de 10 à 30% en poids, par rapport au poids totale du comprimé, d'un excipient approprié pour conférer audit comprimé ses propriétés de muco-adhésion à la paroi vaginale et de libération prolongée, ledit excipient étant constitué d'hydroxypropylméthylcellulose de viscosité dynamique supérieure à 10 000 mPa.s, et l'au moins une souche probiotique et ledit excipient étant comprimés avec une contrainte de compression allant de 50 à 400 MPa.

2. Comprimé selon la revendication 1, dans lequel la souche probiotique est choisie parmi *Lactobacillus rhamnosus, Lactobacillus crispatus, Lactobacillus iners, Lactobacillus gasseri, Lactobacillus jensenii, Lactobacillus acidophilus, Lactobacillus plantarum, Lactobacillus vaginalis, Lactobacillus casei, Lactobacillus reuteri* et leurs combinaisons.

3. Comprimé selon la revendication 2, dans lequel la souche probiotique est *Lactobacillus rhamnosus.*

4. Comprimé selon l'une des revendications précédentes, **caractérisé en ce qu'**il comprend de 10 à 20% en poids dudit excipient par rapport au poids total du comprimé.

5. Comprimé selon l'une des revendications précédentes **caractérisé en ce que** la souche probiotique du genre *Lactobacillus* est sous forme lyophilisée.

6. Comprimé selon l'une des revendications précédentes, **caractérisé en ce qu'**il comprend en outre des cryoprotecteurs, conservateurs, lubrifiants, agents d'écoulement, liants, diluants et leurs combinaisons.

7. Comprimé selon l'une des revendications précédentes pour son utilisation dans la prévention et/ou le traitement des infections vaginales.

8. Comprimé pour son utilisation selon la revendication 7, dans la prévention et/ou le traitement des candidoses.

9. Comprimé pour son utilisation selon la revendication 7, dans la prévention et/ou le traitement des vaginoses.

10. Comprimé pour son utilisation selon l'une des revendications 7 à 9, dans le traitement des candidoses vulvovaginales ou des candidoses vulvovaginales récidivantes.

11. Comprimé pour utilisation selon l'une des revendications 7 à 10 pour une administration locale au plus tous les 2 jours, avantageusement au plus tous les 3 jours, ou tous les 4 jours, ou tous les 5 jours, ou tous les 6 jours ou tous les 7 jours.

12. Procédé de préparation d'un comprimé vaginal muco-adhésif à libération prolongée selon l'une des revendications 1 à 6, **caractérisé en ce qu'**il comprend les étapes suivantes :
a. mélange d'au moins 10⁹ à 10¹¹ UFC par gramme de comprimé d'au moins une souche probiotique du genre *Lactobacillus* avec 10 à 30 % en poids, par rapport au poids total du comprimé de l'hydroxypropylméthylcellulose de viscosité dynamique supérieure à 10 000 mPa.s. appropriée pour conférer audit comprimé ses propriétés de muco-adhésion à la paroi vaginale et de libération prolongée ; et
b. compression du mélange obtenu à l'étape a. avec une contrainte de compression allant de 50 à 400 MPa.

## Patentansprüche

1. Mucoadhäsive Vaginaltablette mit verlängerter Freisetzung, **dadurch gekennzeichnet, dass** sie im Wesentlichen aus 10⁹ bis 10¹¹ CFU mindestens eines probiotischen Stamms der Gattung *Lactobacillus* je Gramm Tablette besteht, komprimiert mit 10 bis 30 Gew.-% im Verhältnis zum Gesamtgewicht der Tablette eines geeigneten Hilfsstoffs, um der Tablette ihre mucoadhäsiven Eigenschaften an der Vaginalwand und der verlängerten Freisetzung zu verleihen, wobei der Hilfsstoff aus Hydroxypropylmethylcellulose mit einer dynamischen Viskosität über 10.000 mPa.s besteht, und der mindestens eine probiotische Stamm und der Hilfsstoff mit einer Kompressionskraft von 50 bis 400 MPa komprimiert sind.

2. Tablette nach Anspruch 1, wobei der probiotische Stamm aus *Lactobacillus rhamnosus, Lactobacillus crispatus, Lactobacillus iners, Lactobacillus gasseri, Lactobacillus jensenii, Lactobacillus acidophilus, Lactobacillus plantarum, Lactobacillus vaginalis, Lactobacillus casei, Lactobacillus reuteri* und deren Kombinationen ausgewählt ist.

3. Tablette nach Anspruch 2, wobei der probiotische Stamm *Lactobacillus rhamnosus* ist.

4. Tablette nach einem der vorangehenden Ansprüche, **dadurch gekennzeichnet, dass** sie 10 bis 20 Gew.-% des Hilfsstoffs im Verhältnis zum Gesamtgewicht der Tablette umfasst.

5. Tablette nach einem der vorangehenden Ansprüche, **dadurch gekennzeichnet, dass** der probiotische Stamm der Gattung *Lactobacillus* in lyophilisierter Form vorliegt.

6. Tablette nach einem der vorangehenden Ansprüche, **dadurch gekennzeichnet, dass** sie ferner Kälteschutzmittel, Konservierungsstoffe, Schmiermittel, Fließmittel, Bindemittel, Verdünner und deren Kombinationen umfasst.

7. Tablette nach einem der vorangehenden Ansprüche für deren Verwendung zur Vorbeugung vor und/oder der Behandlung von Vaginalinfektionen.

8. Tablette für deren Verwendung nach Anspruch 7 zur Vorbeugung vor und/oder der Behandlung von Candidosen.

9. Tablette für deren Verwendung nach Anspruch 7 zur Vorbeugung vor und/oder der Behandlung von Vaginosen.

10. Tablette für deren Verwendung nach einem der Ansprüche 7 bis 9 zur Behandlung von vulvovaginalen Candidosen oder rezidivierenden vulvovaginalen Candidosen.

11. Tablette zur Verwendung nach einem der Ansprüche **7** bis **10** für eine lokale Verabreichung höchstens alle 2 Tage, in vorteilhafter Weise höchstens alle 3 Tage oder alle 4 Tage oder alle 5 Tage oder alle 6 Tage oder alle 7 Tage.

12. Verfahren zur Herstellung einer mucoadhäsiven Vaginaltablette mit verlängerter Freisetzung nach einem der Ansprüche 1 bis 6, **dadurch gekennzeichnet, dass** es die folgenden Schritte umfasst:
a. Mischen von mindestens 10⁹ bis 10¹¹ CFU je Gramm Tablette mindestens eines probiotischen Stamms der Gattung *Lactobacillus* mit 10 bis 30 Gew.-% im Verhältnis zum Gesamtgewicht der Tablette Hydroxypropylmethylcellulose mit einer dynamischen Viskosität über 10.000 mPa.s, die geeignet ist, der Tablette ihre Eigenschaften der Mucoadhäsion an der Vaginalwand und der verlängerten Freisetzung zu verleihen; und
b. Komprimieren des in Schritt a. erhaltenen Gemischs mit einer Kompressionskraft von 50 bis 400 MPa.

## Claims

1. A mucoadhesive sustained-release vaginal tablet, **characterized in that** it is essentially constituted of from 10⁹ to 10¹¹ CFU of at least one probiotic strain of the genus *Lactobacillus* per gram of tablet compressed with from 10 to 30% by weight, relative to the total weight of the tablet, of an excipient suitable for conferring upon said tablet its properties of vaginal wall mucoadhesion and sustained release, said excipient consisting of hydroxypropylmethylcellulose with a dynamic viscosity greater than 10 000 mPa.s, and the at least one probiotic strain and said excipient being compressed with a compressive stress of from 50 to 400 MPa.

2. The tablet according to claim **1,** wherein the probiotic strain is selected from *Lactobacillus rhamnosus, Lactobacillus crispatus, Lactobacillus iners, Lactobacillus gasseri, Lactobacillus jensenii, Lactobacillus acidophilus, Lactobacillus plantarum, Lactobacillus vaginalis, Lactobacillus casei, Lactobacillus reuteri* and combinations thereof.

3. The tablet according to claim **2,** wherein the probiotic strain is *Lactobacillus rhamnosus.*

4. The tablet according to one of the preceding claims, **characterized in that** it comprises from 10 to 20% by weight of said excipient relative to the total weight of the tablet.

5. The tablet according to one of the preceding claims, **characterized in that** the probiotic strain of the genus *Lactobacillus* is in a lyophilized form.

6. The tablet according to one of the preceding claims, **characterized in that** it further comprises cryoprotectants, preservatives, lubricants, flow agents, binders, diluents and combinations thereof.

7. The tablet according to one of the preceding claims, for use in the prevention and/or treatment of vaginal infections.

8. The tablet for use according to claim 7, in the prevention and/or treatment of candidiasis.

9. The tablet for use according to claim 7, in the prevention and/or treatment of vaginosis.

10. The tablet for use according to one of claims 7 to 9, in the treatment of vulvovaginal candidiasis or recurrent vulvovaginal candidiasis.

11. The tablet for use according to one of claims 7 to 10, for local administration at most every 2 days, advantageously at most every 3 days, or every 4 days, or every 5 days, or every 6 days or every 7 days.

12. A method for preparing a mucoadhesive sustained-release vaginal tablet according to one of claims **1** to **6, characterized in that** it comprises the following steps:
a. mixing at least 10⁹ to 10¹¹ CFU per gram of tablet of at least one probiotic strain of the genus *Lactobacillus* with 10 to 30% by weight, relative to the total weight of the tablet, of hydroxypropylmethylcellulose with a dynamic viscosity greater than 10 000 mPa.s suitable for conferring upon said tablet its properties of vaginal wall mucoadhesion and sustained release; and
b. compressing the mixture obtained in step a. with a compressive stress of from 50 to 400 MPa.
